# EUROPEAN PATENT SPECIFICATION

(11) **EP 2 589 371 B1**
(45) Date of publication and mention of the grant of the patent: **25.03.2015**
(21) Application number: 13153271.5
(22) Date of filing: 26.12.2002
(51) Int. Cl.: A61J 3/00, A61M 1/36

(54) **Blood bag system and method of inactivating pathogenic microorganisms**
Blutbeutelsystem und Verfahren zur Inaktivierung pathogener Mikroorganismen
Système de poche de sang et procédé d'inactivation de micro-organismes pathogènes

(30) Priority: 28.12.2001 JP 2001400979
(43) Date of publication of application: 08.05.2013
(62) Divisional of application: 02793416.5
(73) Proprietor: Terumo Kabushiki Kaisha, Tokyo 151-0072 (JP)
(72) Inventor: Sato, Kenichi, Ashigarakami-gun, Kanagawa 259-0151 (JP); Kameyama, Toshiki, Ashigarakami-gun, Kanagawa 259-0151 (JP)
(74) Representative: TBK

(56) References cited:
- CA-A1- 2 165 065
- JP-A- 9 276 368

## Description

The present invention relates to a method of inactivating pathogenic microorganisms in blood and to a blood bag system for supplying microbiologically and toxicologically safe blood preparations.

Blood transfusion is indispensable in modern medicine. Blood transfusion had caused 9,000 people to be infected with HIV until the American National Red Cross commenced screening to remove blood contaminated with HIV (human immunodeficiency virus) in 1985. Thereafter, blood preparations were screened for virus infection and infected blood preparations were discarded. As a result, the number of infected patients by transfusion was reduced to mere 41. Even so, however, it is impossible by the present screening technology to detect pathogenic microorganisms that cause infectious diseases by transfusion such as viruses and bacteria out of inspection items, and thus, the danger of being infected with new virus or the like cannot be excluded. Accordingly, treatment for inactivating pathogenic microorganisms becomes necessary for blood preparations.

Known inactivation technologies for pathogenic microorganisms such as viruses and bacteria thus far developed include Solvent/Detergent (SD) treatment for blood preparations. The treatment has a disadvantage in that it is ineffective to nonenveloped viruses, and safe blood preparations are not necessarily supplied. For this reason, in recent years, inactivators against pathogenic microorganisms are being energetically developed in order to supply safe blood.

For example, virus inactivators containing an ethyleneimine oligomer has been proposed (International Publication Nos. WO97/07674 and WO99/17802). The ethylene oligomer is excellent for inactivation of viruses but has an unstable structure so that it evaporates at ambient temperature and explodes. Accordingly, use of the ethyleneimine oligomer for the inactivation of pathogenic microorganisms in blood preparations involves a high risk of infection of the operator.

Document WO95/00631 describes a continuous-flow or semi-continuous flow system that accomplishes both virus inactivation of blood and virucidal reagent removal from treated blood.

Also, production and storage of the ethyleneimine oligomer requires maintenance at low temperatures, so that the inactivators for pathogenic microorganisms containing an ethyleneimine oligomer not only require much cost for the production facility as well as transportation and storage, but also require further attention for safety management of the inactivators against pathogenic microorganisms containing an ethyleneimine oligomer when used at medical sites.

Therefore, it is an object of the present invention to provide a method of inactivating pathogenic microorganisms in blood free of various problems caused by the conventional inactivators against pathogenic microorganisms for blood preparations in order to supply microbiologically and toxicologically safe blood preparations.

The above objects may be attained by the present invention described below.

The present invention preferably uses a blood bag system including one or more of containers capable of holding a liquid, and a connecting tube connected liquid-tightly to the container, characterized in that: at least one of the containers holds an inactivator that inactivates a pathogenic microorganism contained in blood and/or an anticoagulant; and the inactivator contains as a main component a platinum compound capable of binding to nucleic acids of the microorganism or an aquo complex of the platinum compound.

According to the blood bag system preferably used in the present invention, two or more of the containers are provided, and the inactivator and the anticoagulant are held in different containers, respectively. The container holding the inactivator and the container holding the anticoagulant may be connected by the connecting tube.

Further, according to the blood bag system preferably used in the present invention, the inactivator and the anticoagulant may be held in the same container.

Therefore, the blood bag system preferably used in the present invention is a blood bag system including a blood bag holding an anticoagulant and a container holding an inactivator that inactivates a microorganism contained in blood, connected to each other, in which the inactivator contains as a main component a platinum compound capable of binding to nucleic acid of the microorganism or an aquo complex of the platinum compound; or a blood bag system holding an anticoagulant and an inactivator that inactivates a microorganism contained in blood, in which the inactivator contains as a main component a platinum compound capable of binding to nucleic acid of the microorganism or an aquo complex of the platinum compound.

According to the blood bag system preferably used in the present invention, the platinum compound is preferably at least one selected from the group consisting of cisplatin, carboplatin, and nedaplatin.

According to the blood bag system preferably used in the present invention, the aquo complex of the platinum compound is at least one selected from the group consisting of a monoaquo complex (for example, cis-monochloromonoaqua diammineplatinous(II)chloride), a diaquo complex (for example, cis-diaquodiammineplatinous(II)dinitrate), a monoaquomonohydroxo complex (for example, cis-monohydroxymonoaqua diammineplatinous(II)chloride), and a dihydroxo complex (for example, cis-dihydroxydiammineplatinum(II)).

According to the blood bag system preferably used in the present invention, the pathogenic microorganism is preferably at least one selected from the group consisting of DNA type viruses, RNA type enveloped viruses, and bacteria.

The present invention provides a method of inactivating a pathogenic microorganism in blood as defined in claim 1, including: adding a microorganism inactivator containing as a main component a platinum compound capable of binding to nucleic acids of the microorganism or an aquo complex of the platinum compound to a blood bag that holds blood collected in advance.

According to the method of inactivating a pathogenic microorganism of the present invention, it is preferred that the microorganism inactivator is added so that a concentration becomes 0.07 mM (µmol/mL) to inactivate 1 log₁₀ or more of the pathogenic microorganism in the blood held in the blood bag.

The method of inactivating a pathogenic microorganism of the present invention further includes:
adding a neutralizing agent preferably containing as a main component an amino acid compound or a thiosulfate to neutralize the inactivator, after adding the microorganism inactivator.

According to the method of inactivating a pathogenic microorganism of the present invention, the neutralizing agent is preferably methionine or sodium thiosulfate.

According to the method of inactivating a pathogenic microorganism of the present invention, the neutralizing agent is preferably added so that a concentration becomes 10 to 500 times a concentration of the microorganism inactivator.

Further, the present invention uses an inactivator that inactivates a microorganism contained in blood that is collected in advance containing as an active component a platinum compound or an aquo complex of the platinum compound.

According to the inactivator of the present invention, the platinum compound is preferably at least one selected from the group consisting of cisplatin, carboplatin, and nedaplatin.
Fig. 1 is a schematic diagram illustrating a blood bag system according to one construction example of the present invention;
Fig. 2 is a schematic diagram illustrating a blood bag system according to another construction example of the present invention;
Fig. 3 is a graph illustrating SV-40 killing effect of a platinum compound in Example 1, with the SV-40 killing effect being shown as inactivation effect (logarithm (log₁₀));
Fig. 4 is a graph illustrating SV-40 killing effect of a platinum compound in Example 2, with the SV-40 killing effect being shown as inactivation effect (logarithm (log₁₀));
Fig. 5 is a graph illustrating an effect of cisplatin to inactivate *Yersinia enterocolitica;*
Fig. 6 is a graph illustrating an effect of neutralizing cisplatin in Example 6 with methionine; and
Fig. 7 is a graph illustrating an effect of neutralizing cisplatin in Example 6 with sodium thio sulfate.

Hereinafter, the present invention will be described in detail.

A blood bag system preferably used in the present invention includes at least one container capable of holding a liquid, and a connecting tube liquid-tightly connected to the container capable of holding the liquid for use in charging and discharging the liquid held in the container. At least one of the containers constituting the system holds an anticoagulant in order to hold collected blood. Hereinafter, the container holding the anticoagulant is referred to as a "blood bag". The blood bag system may have, in addition to the blood bag, one or more other containers for use in holding drugs and blood components such as erythrocytes, leukocytes, platelets, or plasma after separation.

Where the blood bag system includes a plurality of containers, the containers are connected to each other with connecting tubes, through which the liquid held in the containers, for example, blood is charged or discharged. Further, the blood bag system may include a connecting tube that is connected to the container at only one end of the connecting tube. Such a tube that is connected to the container at only one end thereof is used mainly for charging and discharging blood between the inside and the outside of the blood bag system. Specific example of such a tube includes a blood collection tube equipped with a needle at an end opposite to the end at which the container is connected.

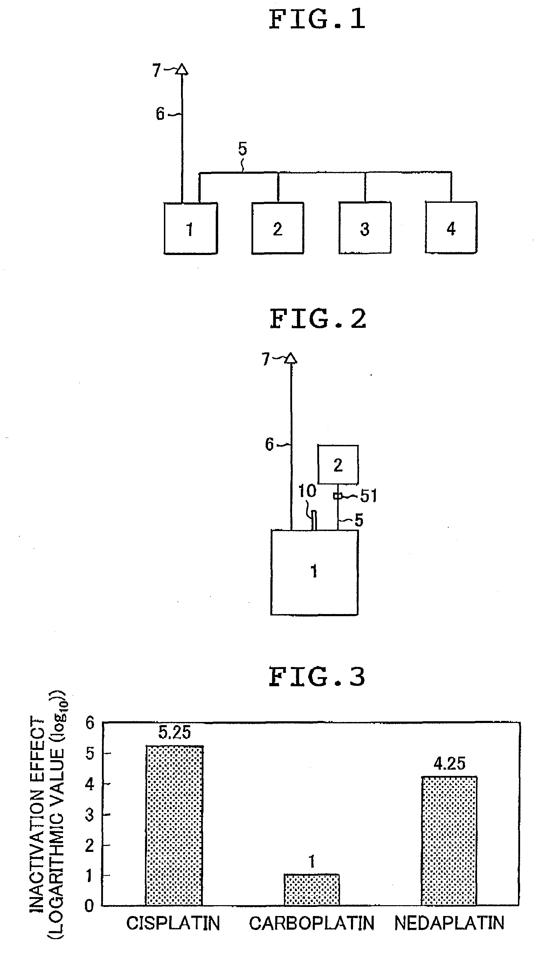

Fig. 1 is a schematic diagram illustrating a blood bag system according to one construction example of the present invention. In the blood bag system illustrated in Fig. 1, a blood bag 1, a container 2 holding an inactivator that inactivates a microorganism contained in blood, and a plurality of (two in Fig. 1) blood component bags 3 and 4 as the other containers are connected through connecting tubes 5. To the blood bag 1 is connected a blood collecting tube 6 equipped with a blood collecting needle 7 at the tip of the tube. The connecting tubes 5 can be sealed by pressing with an aluminum ring or by heat fusion using a heat sealer. Further, the blood component bags 3 and 4 for holding blood components after separation are designed so as to be readily detached separately.

Fig. 2 is a schematic diagram illustrating a blood bag system according to another construction example of the present invention. In the blood bag system shown in Fig. 2, the blood bag 1 and the container 2 holding an inactivator that inactivates a microorganism contained in blood are connected through the connecting tube 5. To prevent migration of the contents between the blood bag 1 and the container 2 unless necessary, the connecting tube 5 is provide with a valve 51 as an opening and closing means for the connecting tube 5. Note that though not shown, in the blood bag system illustrated in Fig. 1, it is preferable that the connecting tubes 5 that connect the blood bag 1, the container 2, and the blood component bags 3 and 4 are provided with a klemme, which is an opening and closing means.

To the blood bag 1 is connected the blood collecting tube 6 equipped with the blood collecting needle 7 at the tip of the tube. Further, the blood bag 1 is provided with an air-bleeding tube 10. When the collected blood from the blood collecting tube 6 or the inactivator from the container 2 is introduced into the blood bag 1, air-bleeding of the air in the inside the blood bag 1 through a tube 10 facilitates the introduction of the blood or inactivator into the blood bag 1.

Each constituent element of the blood bag systems shown in Figs. 1 and 2 are preferably moldings of a thermoplastic resin such as a vinyl chloride resin containing a plasticizer, taking safety into consideration.

In the illustrated blood bag system, the blood bag 1 holds an anticoagulant, so that the coagulation of the collected blood can be prevented. The anticoagulant held in the blood bag 1 may be selected from a wide variety of known anticoagulants. Specific examples of the anticoagulant that can be used include citric acid, heparin, low molecular weight heparin, nafamostat mesilate, gabexate mesilate, and EDTA. The anticoagulants are usually diluted with physiological saline or an aqueous glucose solution and is held in such an amount that the ratio of the anticoagulant liquid to the blood to be held is an ordinary ratio, specifically an amount of, for example, about (1:2 to 1:20). The anticoagulants may constitute a part of a blood preserving liquid. Examples of such a blood preserving liquid include physiological solution such as ACD liquid and CPD liquid that contains one or more of adenine, mannitol, sorbitol, guanosine, and the like.

The container 2 holds an inactivator that inactivates pathogenic microorganisms contained in blood. The inactivator as necessary is introduced into the blood bag 1 through the connecting tube 5 by opening preferably the valve 51, which is an opening and closing means. This inactivates the pathogenic microorganisms contained in the blood in the blood bag 1.

Note that the illustrated blood bag system assumes a construction in which the anticoagulant is held in the blood bag 1 and the inactivator is held in the container 2, that is, a construction in which the anticoagulant and the inactivator are held in different containers. However, the anticoagulant and the inactivator may be held in the same container. That is, in the illustrated construction, the inactivator may be held in advance in the blood bag 1. In this case, inactivation of the pathogenic microorganisms contained in blood is performed as soon as the collected blood is introduced in to the blood bag 1.

However, in the case where a blood preserving liquid containing adenine and guanosine is used, mixing a platinum compound with adenine and guanosine before inactivation of the pathogenic microorganisms must be avoided since adenine and guanosine react with the platinum compound.

According to the present invention, the pathogenic microorganisms contained in blood and that need to be inactivated include DNA-type viruses, RNA enveloped viruses, and bacteria. Viruses are genetically classified into DNA type and RNA type. Structurally, the viruses are classified based on whether or not a viral membrane (envelope) is present. HIV and HCV (human hepatitis C virus) currently causing transfusion infection problems are RNA type enveloped viruses. HBV (human hepatitis B virus) is a DNA type enveloped virus.

Further, bacteria causing transfusion infection problems include *Yersinia enterocolitica* and *Serratia marcescens.* Blood microorganism level of patients infected with these bacteria is 1 × 10¹ to 1 × 10² CFU/mL. Blood collection using a blood bag containing no inactivator results in an increase to 1 × 10⁶ to 1 × 10⁸ CFU/mL in about three weeks. Transfusion of blood having such a microorganism level may sometimes lead to death due to an endotoxin shock.

The present invention is characterized by using a platinum compound known as an anticancer agent, more specifically a platinum compound capable of binding to the nucleic acids of pathogenic microorganisms and/or an aquo complex of the platinum compound (both may be sometimes referred to simply as platinum compound) as a drug that inactivates the pathogenic microorganisms in blood for transfusion (hereinafter, referred to as "inactivator"). When the platinum compound is used as an inactivator, binding of the platinum compound to the nucleic acids of the pathogenic microorganisms is presumed to inactivate the pathogenic microorganisms.

The platinum compound used in the present invention may bind to the nucleic acids of the pathogenic microorganisms. Specific examples thereof include cisplatin, carboplatin, and nedaplatin. Two or more of these may be used in combination.

Further, in the present invention, aquo complexes of the platinum compounds, that is, aquo complexes of cisplatin, carboplatin, nedaplatin, or the like may be used. Preferable aquo complexes include a monoaquo complex such as cis-monochloromonoaqua(II) chloride, a diaquo complex such as cis-diaquodiammineplatinous(II) dinitrate, a monoaquomonohydroxo complex such as cis-monohydroxymonoaqua diammineplatinous(II) chloride, and a dihydroxo complex such as cis-dihydroxydiammineplatinum(II). Two or more of these may be used in combination. Further, these may be used in combination with the above-mentioned platinum compounds.

It is preferable that the platinum compound is dissolved in physiological saline or an aqueous glucose solution and held in the container 2 in a liquid state having an osmotic pressure ratio of about 1 so that it can be introduced from the container 2 to the blood bag 1 through the connecting tube 5, preferably by opening the valve 51 as an opening and closing means.

Further, since the platinum compound is decomposed with light or its stability is changed depending on ion concentration, it is preferable that a light-shielded container is used as the container 2 holding the inactivator or that the ion concentration of the inactivator is adjusted with sodium chloride or the like before the inactivator is held in the container 2.

The method of inactivating pathogenic microorganisms is to inactivate pathogenic microorganisms contained in blood by addition of an inactivator containing as a main component the above-mentioned platinum compound or an aquo complex of the platinum compound to the blood bag holding blood in advance. The method is preferably practiced by using the blood bag system of the present invention as mentioned above. Hereinafter, the method of inactivating pathogenic microorganisms according to the present invention will be described by using the blood bag system illustrated in Fig. 2.

Specifically, in the blood bag system of Fig. 2, the inactivator is introduced from the container 2 into the blood bag 1 that holds blood collected through the blood collecting needle 7 and the blood collecting tube 6 by opening the valve 51 attached to the connecting tube 5, thus practicing the method of inactivating pathogenic microorganisms according to the present invention.

According to the method of the present invention, the amount of platinum compound to be used may vary depending on time, temperature, composition and concentration of blood components and so on. At the time of inactivation, the pathogenic microorganisms in blood held in the blood bag can be inactivated even when a final concentration of the platinum compound in the blood bag 1 is about 0.02 mM (µmol/mL), specifically 0.0209 mM. However, the final concentration of the platinum compound in the blood bag 1 is preferably 0.07 to 100 mM, more preferably 1 to 10 mM. If the final concentration of the platinum compound within the above-mentioned concentration range, the effect of inactivating the pathogenic microorganisms in blood is high and specifically the pathogenic microorganisms in blood can be inactivated by 1 log₁₀ or more. If the final concentration of the platinum compound is above 100 mM, the platinum compound cannot be readily dissolved depending on the solvent so that it is difficult to prepare a solution.

Note that the term "final concentration of platinum compound" as used herein refers to a concentration (mM) of a platinum compound at the time when substantially all of the platinum compound to be used is introduced into the blood bag 1 upon the inactivation treatment.

Specifically showing the amount of the platinum compound to be used in the method of the present invention, when the platinum compound is cisplatin and the pathogenic microorganisms in a concentrated erythrocyte preparation (RC-MAP) are to be inactivated, the platinum compound is used in an amount so that a final concentration becomes 21 µg/mL to 30 mg/mL, preferably 0.3 mg/mL to 3 mg/mL.

Note that the effective concentration of cisplatin against cancer cells is 0.25 µg/mL (antiviral activity (IC₅₀) on initial culture cells of Ehrlich cancer), and the above-mentioned final concentration of cisplatin of 21 µg/mL is as high as 84 times. That is, in the case of blood preparations, cisplatin is used in concentrations much higher than the concentration in which it is used as an anticancer agent to humans.

According to the method of the present invention, the temperature at which the platinum compound is added to blood for a reaction between the platinum compound and the nucleic acids of pathogenic microorganisms only needs to be 0°C or higher but is preferably 4 to 30°C in consideration of influences on blood preparations.

Also, according to the present invention, upon addition of the platinum compound to blood, the reaction between the platinum compound and the nucleic acids of pathogenic microorganisms starts at once. The reaction is preferably continued for 3 hours or more in order to effect the inactivation of the pathogenic microorganisms more completely.

Since the platinum compound used in the method of the present invention belongs to poison in toxicology and since the platinum compound are used in very high concentration as mentioned above, neutralization treatment is preferably performed after the inactivation treatment in order to alleviate the toxicity of the platinum compound. In such neutralization treatment, a neutralizing agent that is usually used in converting the toxicity of anticancer agents containing the platinum compounds to toxicologically safe is preferably added to blood preparations containing the platinum compound after inactivation. Also, the neutralization treatment may be performed by irradiation of light. Note that the neutralization treatment also involves decreasing the reactivity of the platinum compound with the nucleic acids of the pathogenic microorganisms, and thus, can serve to terminate the inactivation reaction.

To add the neutralizing agent to blood preparations containing the platinum compound after inactivation reaction for a desired period of time, for example, a container containing a neutralizing agent is connected in advance to the blood bag 1 through a connecting tube having a valve as an opening and closing means and the valve is opened as necessary to introduce the neutralizing agent into the blood bag 1. Also, a neutralizing agent may be introduced into the blood bag 1 from the air-bleeding tube 10 by using a syringe or the like.

A reaction of the neutralizing agent with the platinum compound, as a microorganism inactivator, in a concentration 42 times or higher the concentration of the platinum compound can result in complete neutralization treatment. Usually, the neutralizing agent is added in a concentration 10 to 500 times, preferably 25 to 100 times the concentration of the platinum compound.

Although the neutralizing treatment is effective at a temperature of 0°C or higher, the reaction is preferably performed at a temperature of 4 to 30°C in consideration of influences on the function of blood preparations.

Further, although the reaction starts immediately after addition of the platinum compound, the reaction is preferably continued for about 30 minutes to about 1 hour in order to ensure the neutralization treatment.

Examples of the neutralizing agent for cisplatin include thiosulfates such as sodium thiosulfate, amino acids such as methionine and cysteine, and thiol compounds such as glutathione and coenzyme A, with sodium thiosulfate and methionine being preferable. To conveniently introduce the neutralizing agent into the blood bag 1, the neutralizing agent is preferably liquid added in a state having an osmotic pressure ratio of about 1 after dissolving the neutralizing agent in physiological saline or an aqueous glucose solution.

Note that photodegradable neutralizing agent such as methionine is stored preferably in a light-shielding container.

The method of inactivating pathogenic microorganisms according to the present invention by preferably using the blood bag systems illustrated in Figs. 1 and 2 is described above, the method of the present invention is not limited thereto. For example, in the case where the blood bag system of the present invention holds an anticoagulant and an inactivator in the blood bag in advance, the inactivation reaction of the pathogenic microorganisms in blood starts at the time when the collected blood is introduced into the blood bag. Further, the method of the present invention may be performed by introducing the inactivator into the blood bag that holds blood in advance by using a syringe or the like.

Hereinafter, the present invention will be described in detail by examples. However, the present invention should not be considered as being limited thereto.

### (Example 1)

### [Virus inactivation for simian virus 40 (SV-40) which is a DNA type nonenveloped virus]

In a 400-mL CPDA blood bag in the blood bag system shown in Fig. 1, RC-MAP (concentrated erythrocyte preparation) was prepared from blood collected from healthy humans. Thereafter, 2 mL of RC-MAP was sampled in a 15-mL centrifuging tube aseptically, and 2 mL of 10⁸ TCID₅₀/mL (50% infection end point) SV-40 was added thereto, followed by gentle stirring. To the resultant was added 2 mL of a cisplatin solution containing 1.67 mM cisplatin in an aqueous solution containing sodium chloride and hydrochloric acid (Briplatin (registered trademark)) as an inactivator, followed by gently stirring and a reaction at room temperature (in the dark) for 7 hours to effect inactivation. After completion of the inactivation, the inactivated solution was centrifuged at 3,000 rpm for 1 minute and the supernatant was used for the evaluation of virus inactivation.

Further, inactivation was similarly performed using, instead of the cisplatin solution, a 3.5 mM carboplatin solution having dissolved carboplatin (Paraplatin (registered trademark)) in a phosphate buffer and a 3.5 mM nedaplatin solution having dissolved nedaplatin (Aqupla (registered trademark) in a phosphate buffer as inactivators and the supernatants of centrifuged inactivated solutions were used for the evaluation of virus inactivation.

Evaluation of virus inactivation was performed by TCID₅₀ using a 96-well microplate. In a 25-cm² cell culture flask, a CV-1 cell (simian kidney derived cell), which is a proliferating cell of SV-40, was cultivated in a 10% fetal bovine serum-added MEM medium for 3 days for proliferation. Thereafter, the cells were floated with 0.5 mL of 0.05% trypsin-EDTA, to prepare a cell suspension in 25 mL of the 10% fetal bovine serum-added MEM medium.

After 90 µL of the cell suspension was added to each well of the 96-well microplate, 10 µL of the reaction mixture (the above-mentioned supernatant) was added to each well of the first column of the 96-well microplate and pipetting was performed to stir the resultant. Further, 10 µL of the solution in each well of the first column was added to each well of the second column of the microplate to perform 10-fold gradual dilution operation. This operation was repeated up to the wells of the twelfth column of the microplate. The microplate was incubated in a 5% carbon dioxide gas incubator at 37°C for 10 days. Then, a cytopathic effect (CPE) by SV-40 was observed using an optical microscope, and TCID₅₀ were calculated as logarithmic values (log₁₀).

At the same time, TCID₅₀ of a positive control, in which a phosphate buffer was added instead of the platinum compound, was calculated as a logarithmic value (log₁₀). A value obtained by subtracting TCID₅₀ of the inactivated solution from TCID₅₀ of the positive control was defined as a killing effect.

The results are shown in Fig. 3. In Fig. 3, the inactivation effect means a value obtained by subtracting TCID₅₀ of the inactivated solution (logarithmic value (log₁₀)) from TCID₅₀ of the above-mentioned positive control (logarithmic value (log₁₀)).

Here, TCID₅₀ of the positive control was 6.5 log₁₀ and TCID₅₀ of the inactivated solutions obtained by reacting with the cisplatin solution, carboplatin solution, and nedaplatin solution, respectively, as the inactivators were 1.25 log₁₀, 5.50 log₁₀, and 2.25 log₁₀, respectively. The results obtained by subtracting the latter from the former confirmed that the 1.67 mM cisplatin solution had an inactivation effect of 5.25 log₁₀, the 3.5 mM carboplatin solution had an inactivation effect of 1.00 log₁₀, and the 3.5 mM nedaplatin solution had an inactivation effect of 4.25 log₁₀.

### (Example 2)

### [Virus inactivation for simian virus 40 (SV-40) which is a DNA type nonenveloped virus using a blood bag system of the present invention]

Blood was collected from healthy humans and RC-MAP (concentrated erythrocyte preparation) was prepared, which was introduced into the blood bag 1 (400-mL CPDA blood bag) in the blood bag system shown in Fig. 2. Thereafter, 10⁸ TCID₅₀/mL (50% infection endpoint) of SV-40 per 2 mL of RC-MAP was added to the blood bag 1 from the air-bleeding tube 10 by using a syringe, and the blood bag 1 was gently stirred.

Then, by opening the valve 51, a cisplatin solution containing 1.67 mM (µmol/mL) cisplatin in an aqueous solution containing sodium chloride and saline (Briplatin (registered trademark), available from Bristol Myers Squibb company) was introduced into the blood bag 1 in an amount of 2 mL per 2 mL of RC-MAP. Thereafter, the blood bag was stirred gently, and the resultant was allowed to react at room temperature (in the dark) for 7 hours to effect inactivation. After completion of the inactivation, 4 mL of the inactivated solution was sampled from the blood bag 1 and centrifuged at 3,000 rpm for 1 minute. The supernatant was used for the evaluation of virus inactivation.

Further, inactivation was similarly performed using, instead of the cisplatin solution, a 3.5 mM carboplatin solution having dissolved carboplatin (Paraplatin (registered trademark), Bristol Pharmaceuticals K.K.) in a phosphate buffer and a 3.5 mM nedaplatin solution having dissolved nedaplatin (Aqupla (registered trademark), Shionogi & Co., Ltd.) in a phosphate buffer as inactivators and the supernatants of centrifuged inactivated sampled from the blood bag 1 were used for the evaluation of virus inactivation.

Evaluation of virus inactivation was performed by TCID₅₀ using a 96-well microplate. In a 25-cm² cell culture flask, a CV-1 cell (simian kidney derived cell), which is a proliferating cell of SV-40, was cultivated in a 10% fetal bovine serum-added MEM medium for 3 days for proliferation. Thereafter, the cells were floated with 0.5 mL of 0.05% trypsin-EDTA, to prepare a cell suspension in 25 mL of the 10% fetal bovine serum-added MEM medium.

After 90 µL of the cell suspension was added to each well of the 96-well microplate, 10 µL of the reaction mixture (the above-mentioned supernatant) was added to each well of the first column of the 96-well microplate and pipetting was performed to stir the resultant. Further, 10 µL of the solution in each well of the first column was added to each well of the second column of the microplate to perform 10-fold gradual dilution operation. This operation was repeated up to the wells of the twelfth column of the microplate. The microplate was incubated in a 5% carbon dioxide gas incubator at 37°C for 10 days. Then, a cytopathic effect (CPE) by SV-40 was observed using an optical microscope, and TCID₅₀ were calculated as logarithmic values (log₁₀).

At the same time, TCID₅₀ of a positive control, in which a phosphate buffer was added instead of the platinum compound, was calculated as a logarithmic value (log₁₀). A value obtained by subtracting TCID₅₀ of the inactivated solution from TCID₅₀ of the positive control was defined as a killing effect (inactivation effect).

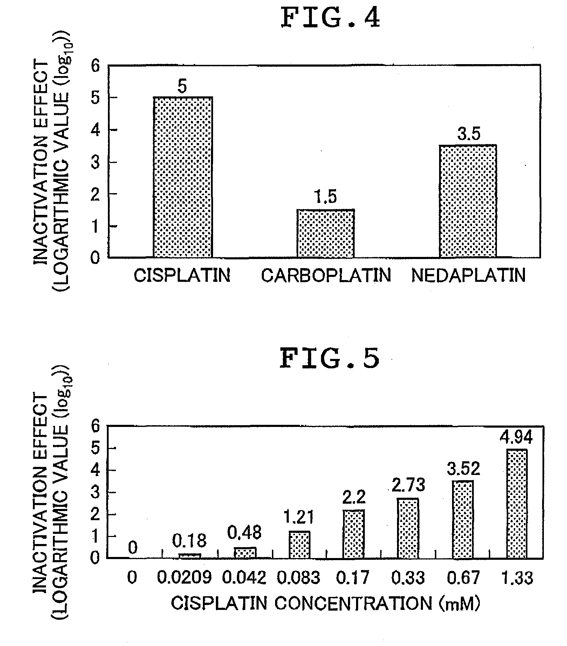

The results are shown in Fig. 4. In Fig. 4, the inactivation effect means a value obtained by subtracting TCID₅₀ of the inactivated solution (logarithmic value (log₁₀)) from TCID₅₀ of the above-mentioned positive control (logarithmic value (log₁₀)).

Here, TCID₅₀ of the positive control was 6.0 log₁₀ and TCID₅₀ of the inactivated solutions obtained by reacting with the cisplatin solution, carboplatin solution, and nedaplatin solution, respectively, as the inactivators were 1.0 log₁₀, 4.5 log₁₀, and 2.5 log₁₀, respectively. As the results obtained by subtracting the latter from the former, Fig. 4 shows that the 1.67 mM cisplatin solution had an inactivation effect of 5.0 log₁₀, the 3.5 mM carboplatin solution had an inactivation effect of 1.5 log₁₀, and the 3.5 mM nedaplatin solution had an inactivation effect of 3.5 log₁₀.

### (Example 3)

### [Virus inactivation for Human cytomegalovirus (HCMV) which is a DNA type enveloped virus]

In the blood bag 1 (a 400-mL CPDA blood bag) in the blood bag system shown in Fig. 1, RC-MAP (concentrated erythrocyte preparation) was prepared from blood collected from healthy humans. Thereafter, 2 mL of RC-MAP was sampled in a 15-mL centrifuging tube aseptically, and 2 mL of 10⁷ TCID₅₀/mL HCMV was added thereto, followed by gentle stirring. To the resultant was added 2 mL of a cisplatin solution containing 1.67 mM cisplatin in an aqueous solution containing sodium chloride and hydrochloric acid (Briplatin (registered trademark)) as an inactivator, followed by gently stirring and a reaction at room temperature (in the dark) for 7 hours to effect inactivation. After completion of the inactivation, the inactivated solution was centrifuged at 3,000 rpm for 1 minute and the supernatant was used for the evaluation of virus inactivation.

Evaluation of virus inactivation was performed by TCID₅₀ using a 96-well microplate. In a 25-cm² cell culture flask, a HEL cell (primary human embryonic lung fibroblasts cell), which is a proliferating cell of HCMV, was cultivated in a 10% fetal bovine serum-added MEM medium for 3 days for proliferation. Thereafter, the cells were floated with 0.5 mL of 0.05% trypsin-EDTA, to prepare a cell suspension in 25 mL of the 10% fetal bovine serum-added MEM medium.

After 90 µL of this cell suspension was added to each well of the 96-well microplate, 10 µL of the solution (the above-mentioned supernatant) was added to each well of the first column of the 96-well microplate and pipetting was performed to stir the resultant. Further, 10 µL of the solution in each well of the first column was added to each well of the second column of the microplate to perform 10-fold gradual dilution operation. This operation was repeated up to the wells of the twelfth column of the microplate. The microplate was incubated in a 5% carbon dioxide gas incubator at 37°C for 14 days. Then, a cytopathic effect (CPE) by HCMV was observed using an optical microscope, and TCID₅₀ was calculated as a logarithmic value (log₁₀). At the same time, TCID₅₀ of a positive control, in which a phosphate buffer was added instead of the platinum compound, was calculated as a logarithmic value (log₁₀). A value obtained by subtracting TCID₅₀ of the inactivated solution from TCID₅₀ of the positive control was defined as an anti-HCMV effect of cisplatin (inactivation effect).

Here, TCID₅₀ of the positive control was 5.25 log₁₀ and TCID₅₀ of the inactivated solution obtained by reacting with the cisplatin solution was 1.25 log₁₀. The results obtained by subtracting the latter from the former confirmed that the 1.67 mM cisplatin solution killed 4.00 log₁₀ of HCMV in RC-MAP.

### (Example 4)

### [Virus inactivation for mouse hepatitis virus (MHV) which is an RNA type enveloped virus]

In the blood bag 1 (a 400-mL CPDA blood bag) in the blood bag system shown in Fig. 1, RC-MAP (concentrated erythrocyte preparation) was prepared from blood collected from healthy humans. Thereafter, 2 mL of RC-MAP was sampled in a 15-mL centrifuging tube aseptically, and 2 mL of 10⁷ TCID₁₀/mL MHV was added thereto, followed by gentle stirring. To the resultant was added 2 mL of a cisplatin solution containing 1.67 mM cisplatin in an aqueous solution containing sodium chloride and hydrochloric acid (Briplatin (registered trademark)) as an inactivator, followed by gently stirring and a reaction at room temperature (in the dark) for 7 hours to effect inactivation. After completion of the inactivation, the inactivated solution was centrifuged at 3,000 rpm for 1 minute and the supernatant was used for the evaluation of virus inactivation.

Evaluation of virus inactivation was performed by TCID₅₀ using a 96-well microplate. In a 25-cm² cell culture flask, a DBT cell, which is a proliferating cell of MHV, was cultivated in a 5% fetal bovine serum-added MEM medium for 3 days for proliferation. Thereafter, the cells were floated with 0.5 mL of 0.05% trypsin-EDTA, to prepare a cell suspension in 25 mL of the 10% fetal bovine serum-added MEM medium.

After 90 µL of this cell suspension was added to each well of the 96-well microplate, 10 µL of the solution was added to each well of the first column of the 96-well microplate and pipetting was performed to stir the resultant. Further, 10 µL of the solution in each well of the first column was added to each well of the second column of the microplate to perform 10-fold gradual dilution operation. This operation was repeated up to the wells of the twelfth column of the microplate. The microplate was incubated in a 5% carbon dioxide gas incubator at 37°C for 14 days. Then, a cytopathic effect (CPE) by MHV was observed using an optical microscope, and TCID₅₀ was calculated as a logarithmic value (log₁₀). At the same time, TCID₅₀ of a positive control, in which a phosphate buffer was added instead of the platinum compound, was calculated as a logarithmic value (log₁₀). A value obtained by subtracting TCID₅₀ of the inactivated solution from TCID₅₀ of the positive control was defined as an MHV inactivation effect.

Here, TCID₅₀ of the positive control was 5.50 log₁₀ and TCID₅₀ of the inactivated solution obtained by reacting with the cisplatin solution was 1.25 log₁₀. The result obtained by subtracting the latter from the former confirmed that the 1.67 mM cisplatin solution killed 4.00 log₁₀ of MHV in RC-MAP.

### (Example 5)

### [Bacteria inactivation of Yersinia enterocolitica (Yersinia strain)]

In the blood bag 1 (400-mL CPDA blood bag) of the blood bag system shown in Fig. 1, RC-MAP (concentrated erythrocyte preparation) was prepared from blood collected from healthy humans. Thereafter, 2 mL of RC-MAP was aseptically sampled in a 15-mL centrifuging tube, and 2 mL of 10⁸ CFU/mL *Yersinia enterocolitica* was added thereto, followed by gently stirring. To the resultant was added 2 mL of a cisplatin solution having dissolved cisplatin (available from Wako Pure Chemical Industries, Ltd.) in physiological saline and adjusted to a concentration of 4.00 mM, as an inactivator. After gentle stirring, the resultant was allowed to react at room temperature (in the dark) for 7 hours to effect inactivation. After completion of the inactivation, the inactivated solution was centrifuged at 3,000 rpm for 1 minute and the supernatant was used for the evaluation of virus inactivation.

Similarly, cisplatin solutions having dissolved cisplatin (available from Wako Pure Chemical Industries, Ltd.) in physiological saline and adjusted to concentrations of 2.00 mM, 1.00 mM, 0.50 mM, 0.25 mM, 0.125 mM, and 0.0625 mM were used as inactivators to effect inactivation, and the supernatants were used for the evaluation of bacteria inactivation.

Evaluation of antibacterial activity was performed on 10-fold gradually diluted solutions obtained by adding 1 mL of the inactivated solution to 9 mL of sterilized physiological saline, stirring the mixture by using a touch mixer, and further adding 1 mL of the resultant to 9 mL of physiological saline for dilution. 1 mL or 100 µL of the diluted solution in each stage was mixed in a petri dish having a diameter of 9 cm and containing 15 mL soybean casein digest agar medium. This was cultivated at 31°C for 2 days. The number of colonies emerged was defined as number of survival bacteria.

Note that solution to which no cisplatin was added was defined as a positive control. A bacteria inactivation effect was defined by a value obtained by subtracting the number of survival bacteria (logarithmic value (log₁₀)) in cisplatin-added inactivated solution from the number of survival bacteria (logarithmic value (log₁₀)) in the positive control.

The results are shown in Fig. 5. Here, the number of survival bacteria was 5.00 log₁₀, and the numbers of survival bacteria in the inactivated solutions inactivated by the cisplatin solutions are as follows.

| Concentration of Cisplatin | Number of survival bacteria |
|---|---|
| 1.33 mM | 0.06 log₁₀ |
| 0.67 mM | 1.48 log₁₀ |
| 0.33 mM | 2.27 log₁₀ |
| 0.17 mM | 2.80 log₁₀ |
| 0.083 mM | 3.79 log₁₀ |
| 0.042 mM | 4.52 log₁₀ |
| 0.0209 mM | 4.82 log₁₀ |

As a result of subtracting the latter from the former, the bacteria killing effect of each inactivator was as follows.

| Concentration of cisplatin in inactivator | Bacteria killing effect |
|---|---|
| 1.33 mM | 4.99 log₁₀ |
| 0.67 mM | 3.52 log₁₀ |
| 0.33 mM | 2.73 log₁₀ |
| 0.17 mM | 2.20 log₁₀ |
| 0.083 mM | 1.21 log₁₀ |
| 0.042 mM | 0.48 log₁₀ |
| 0.0209 mM | 0.18 log₁₀ |

### (Example 6)

### [Detoxication of cisplatin/anti-Yersinia enterocolitica activity after reaction of cisplatin with sodium thiosulfate or methionine]

In the blood bag 1 (400-mL CPDA blood bag) of the blood bag system shown in Fig. 1, RC-MAP (concentrated erythrocyte preparation) was prepared from blood collected from healthy humans. Thereafter, 2 mL of RC-MAP was aseptically sampled in a 15-mL centrifuging tube and 2mL of a cisplatin solution having dissolved cisplatin (available Wako Pure Chemical Industries, (Ltd.)) in physiological saline and adjusted to a concentration of 4.00 mM as an inactivation was added, followed by gentle stirring. To the resultant was added 2 mL of a 168 mM L-methionine solution having dissolved L-methionine in physiological saline, for a reaction at room temperature for 30 minutes.

Similarly, as neutralizing agents, L-methionine solutions having concentrations of 84 mM, 42 mM, 21 mM, 10.5 mM, 5.25 mM, 2.63 mM, and 1.31 mM, respectively, obtained by dissolving L-methionine in physiological saline or sodium thiosulfate solutions having concentrations of 168 mM, 84 mM, 42 mM, 21 mM, 10.5 mM, 5.25 mM, 2.63 mM, and 1.31 mM, respectively, obtained by dissolving sodium thiosulfate in physiological saline were used to effect a reaction. In addition, solutions to which a phosphate buffer was added instead of the inactivator and neutralizing agent (without cisplatin) were prepared as controls, and the reaction was performed in the similar manner. Thereafter, 10 µL of 10⁸ CFU/mL *Yersinia enterocolitica* was added to each solution, and the number of bacterial cells after stirring was measured.

Measurement of bacterial cells was conducted on solutions obtained by repeating 3 times the 10-fold gradual dilution operation consisting of adding 1 mL of the inactivated solution to 9 mL of sterilized physiological saline, stirring the mixture by using a touch mixer, and further adding 1 mL of the resultant to 9 mL of physiological saline for dilution. 10 mL, 1 mL or 100 µL of the diluted solution in each stage was mixed in a petri dish having a diameter of 9 cm and containing 15 mL soybean casein digest agar medium. This was cultivated at 31°C for 2 days. The number of colonies emerged was defined as number of survival bacteria, and a logarithmic value (log₁₀) thereof was defined as a neutralization effect.

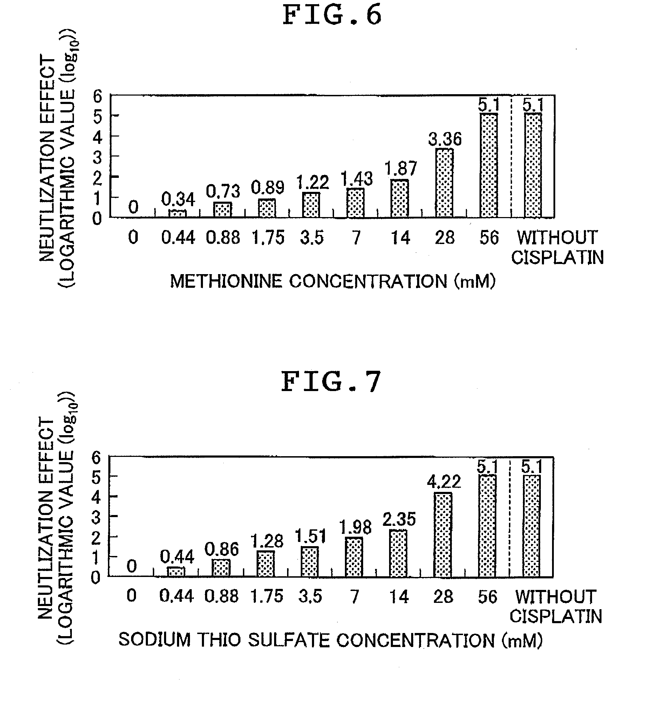

The results of neutralization treatment with methionine as a neutralizing agent are shown in Fig. 6. The results confirmed that in the case where a neutralizing agent having a methionine concentration of 56 mM showed the same result as that obtained without cisplatin (number of survival bacteria of 5.1 log₁₀) and *Yersinia enterocolitica* were not killed at all. In other cases where the neutralizing agent was used in different concentrations, the numbers of survival bacteria were as follows.

| Concentration of methionine in neutralizing agent | Number of survival bacteria |
|---|---|
| 28 mM | 3.36 log₁₀ |
| 14 mM | 1.87 log₁₀ |
| 7 mM | 1.43 log₁₀ |
| 3.5 mM | 1.22 log₁₀ |
| 1.75 mM | 0.89 log₁₀ |
| 0.88 mM | 0.73 log₁₀ |
| 0.44 mM | 0.34 log₁₀ |

On the other hand, the results of the neutralization treatment with sodium thiosulfate as a neutralizing agent are shown in Fig. 7. The results confirmed that in the case where a neutralizing agent having a sodium thiosulfate concentration of 56 mM showed the same result as that obtained without cisplatin (number of survival bacteria of 5.1 log₁₀) and *Yersinia enterocolitica* were not killed at all. In other cases where the neutralizing agent was used in different concentrations, the numbers of survival bacteria were as follows.

| Concentration of sodium thiosulfate in neutralizing agent | Number of survival bacteria |
|---|---|
| 28 mM | 4.22 log₁₀ |
| 14 mM | 2.35 log₁₀ |
| 7 mM | 1.98 log₁₀ |
| 3.5 mM | 1.51 log₁₀ |
| 1.75 mM | 1.28 log₁₀ |
| 0.88 mM | 0.86 log₁₀ |
| 0.44 mM | 0.44 log₁₀ |

### (Example 7)

### [Neutralization treatment of cisplatin/anti-SV-40 activity after reaction of cisplatin with sodium thiosulfate or methionine]

In the blood bag 1 (400 mL CPDA blood bag) of the blood bag system shown in Fig. 1, RC-MAP (concentrated erythrocyte preparation) was prepared from blood collected from healthy humans. Thereafter, 1 mL of RC-MAP was sampled in a 15-mL centrifuging tube aseptically and 1 mL of a cisplatin solution having dissolved cisplatin (available from Wako Pure Chemical Industries, Ltd.) in physiological saline and adjusted to a concentration of 3.5 mM cisplatin was added thereto as an inactivator, followed by gentle stirring. To the resultant was added 1 mL of a 175 mM sodium thiosulfate solution having dissolved sodium thiosulfate in physiological saline, for a reaction at room temperature for 30 minutes. Thereafter, 0.5 mL of 10⁸ TCID5₅₀/mL SV-40 was added and the resultant was stirred gently and then allowed to react in an incubator (in the dark) at room temperature for 30 minutes. After completion of the reaction, the resultant mixture was centrifuged at 3,000 rpm for 1 minute and the supernatant was used for the evaluation of virus inactivation.

Evaluation of virus inactivation was performed by TCID₅₀ using a 96-well microplate. In a 25-cm² cell culture flask, a CV-1 cell (simian kidney derived cell), which is a proliferating cell of SV-40, was cultivated in a 10% fetal bovine serum-added MEM medium for 3 days for proliferation. Thereafter, the cells were floated with 0.5 mL of 0.05% trypsin-EDTA, to prepare a cell suspension in 25 mL of the 10% fetal bovine serum-added MEM medium.

After 90 µL of the cell suspension was added to each well of the 96-well microplate, 10 µL of the reaction mixture (the above-mentioned supernatant) was added to each well of the first column of the 96-well microplate and pipetting was performed to stir the resultant. Further, 10 µL of the solution in each well of the first column was added to each well of the second column of the microplate to perform 10-fold gradual dilution operation. This operation was repeated up to the wells of the twelfth column of the microplate. The microplate was incubated in a 5% carbon dioxide gas incubator at 37°C for 10 days. Then, a cytopathic effect (CPE) by SV-40 was observed using an optical microscope, and TCID₅₀ was calculated as a logarithmic value (log₁₀). At the same time, TCID₅₀ of a positive control, in which a phosphate buffer was added instead of the platinum compound was calculated as a logarithmic value (log₁₀). A value obtained by subtracting TCID₅₀ of the inactivated solution from TCID₅₀ of the positive control was defined as a killing effect.

As a result, subtraction of TCID₅₀ (6.00 log₁₀) of the case where a cisplatin solution having a concentration of 3.5 mM as an inactivator and then a sodium thiosulfate solution having a concentration of 175 mM as a neutralizing agent were added from TCID₅₀ (6.00 log₁₀) of the positive control indicates no killing effect, that is, inactivation effect of SV-40. This means that the inactivator containing cisplatin in a concentration of 3.5 mM was neutralized by addition of the neutralizing agent containing sodium thiosulfate in a concentration of 175 mM.

### (Example 8)

### [Hemolysis toxicity of cisplatin on erythrocyte preparation]

In the blood bag 1 (400-mL CPDA blood bag) of the blood bag system shown in Fig. 1, RC-MAP (concentrated erythrocyte preparation) was prepared from blood collected from healthy humans. Thereafter, 1 mL of RC-MAP was aseptically sampled in a 15-mL centrifuging tube, and 1 mL of a cisplatin solution having dissolved cisplatin (Briplatin (registered trademark)) in physiological saline in a concentration of 1.67 mM and 1 mL of physiological saline were added thereto while gently mixing. The resultant was allowed to react at room temperature (in the dark) for 7 hours. Thereafter, the resultant mixture was centrifuged at 2,000 rpm for 5 minutes. Drabkin's reagent was added to the supernatant for a reaction and absorbance at 540 nm was measured to determine hemolysis rate by a cyanmethemoglobin method. As a result, the hemolysis rate of cisplatin was found to be 0.1% or less, confirming that cisplatin has low hemolysis toxicity on erythrocyte preparations.

### INDUSTRIAL APPLICABILITY

The present invention employs a platinum compound that is used as an anticancer agent and whose characteristics are widely known as an inactivator for pathogenic microorganisms in blood, and thus, the present invention is safe and the inactivator is easily available. Further, since there is no need to newly develop inactivators, the present invention is excellent in view of cost.

Further, use of the platinum compound in the present invention in a concentration as high as 0.07 mM or more, which is much higher than the amount of the platinum compound used as original use of anticancer agent, increases an effect of inactivating pathogenic microorganisms and provides an excellent sustained inactivation effect.

In the present invention, transfusion of inactivated blood after neutralization treatment of the anticancer agent and washing enables supply of microbiologically and toxicologically safe blood preparations unlike the original usage of anticancer agents used for the treatment of cancers.

In the present invention, blood or blood components containing inactivated pathogenic microorganisms and preferably further neutralized are held in bags, which are separated into individual bags in use for transfusion. Upon storage and transportation, methods and means that are applied to conventional bags can be applied as they are.

This application is a divisional application of European patent application no. 02793416.5 (the "parent application"), also published under no. EP 1459724. The content of the original claims of the parent application is repeated below in the present description and form part of the content of this description as follows:

### ITEMS

1. A blood bag system comprising one or more of containers capable of holding a liquid, and a connecting tube connected liquid-tightly to the container, characterized in that:
   at least one of the containers holds an inactivator that inactivates a pathogenic microorganism contained in blood and/or an anticoagulant; and
   the inactivator contains as a main component a platinum compound capable of binding to nucleic acids of the microorganism or an aquo complex of the platinum compound.
2. A blood bag system according to item 1 further comprising two or more of the containers,
   wherein the inactivator and the anticoagulant are held in different containers, and
   wherein the container holding the inactivator and the container holding the anticoagulant are connected by the connecting tube.
3. A blood bag system according to item 1 or 2, wherein the platinum compound is at least one selected from the group consisting of cisplatin, carboplatin, and nedaplatin.
4. A blood bag system according to item 1 or 2, wherein the aquo complex of the platinum compound is at least one selected from the group consisting of a monoaquo complex, a diaquo complex, a monoaquomonohydroxo complex, and a dihydroxo complex.
5. A blood bag system according to any one of items 1 to 4, wherein the pathogenic microorganism is at least one selected from the group consisting of DNA type viruses, RNA type enveloped viruses, and bacteria.
6. A method of inactivating a pathogenic microorganism in blood, comprising:
   adding a microorganism inactivator containing as a main component a platinum compound capable of binding to nucleic acids of the microorganism or an aquo complex of the platinum compound to a blood bag that holds blood collected in advance.
7. A method of inactivating a pathogenic microorganism according to item 6, wherein the microorganism inactivator is added so that a concentration becomes 0.07 mM (µmol/mL) to inactivate 1 log₁₀ or more of the pathogenic microorganism in the blood held in the blood bag.
8. A method of inactivating a pathogenic microorganism according to item 6 or 7, further comprising adding a neutralizing agent containing as a main component an amino acid compound or a thiosulfate to neutralize the inactivator, after adding the microorganism inactivator.
9. A method of inactivating a pathogenic microorganism according to item 8, wherein the neutralizing agent is methionine or sodium thiosulfate.
10. A method of inactivating a pathogenic microorganism according to item 8 or 9, wherein the neutralizing agent is added so that a concentration becomes 10 to 500 times a concentration of the microorganism inactivator.

## Claims

1. A method of inactivating a pathogenic microorganism by using a blood bag system which comprises a blood bag (1) holding an anticoagulant, a container (2) holding an inactivator that inactivates a microorganism contained in blood, and a connecting tube (5) connected liquid-tightly to the container (2), wherein the inactivator contains as a main component a platinum compound capable of binding to nucleic acid of the microorganism or an aquo complex of the platinum compound;; the method comprises
a step of introducing blood into the blood bag (1),
a step of introducing the inactivator into the blood bag (1) through the connecting tube (5) from the container (2), and
**characterized by**
a step of introducing a neutralizing agent into the blood bag (1)to neutralize the inactivator .

2. The method according to claim 1, wherein the step of introducing the inactivator into the blood bag (1) through the connecting tube (5) from the container (2) is followed by the step of introducing blood into the blood bag (1).

3. The method according to claim 1, wherein the connecting tube (5) is provided with a valve (51), the valve (51) is closed in the step of introducing blood into the blood bag (1), the valve (51) is opened in the step of introducing the inactivator into the blood bag (1) through the connecting tube (5) from the container (2).

4. The method according to claim 2, wherein the connecting tube (5) is provided with a valve (51), the valve (51) is closed in the step of introducing blood into the blood bag (1) and in the step of introducing the inactivator into the blood bag (1) through the connecting tube (5) from the container (2), the valve (51) is opened after completion of the step of introducing blood into the blood bag (1) and in the step of introducing the inactivator into the blood bag (1) through the connecting tube (5) from the container (2).

5. The method according to any one of the preceding claims, wherein the blood bag system is provided with a container containing the neutralizing agent which is connected to the blood bag (1) through a connecting tube having a valve as an opening and closing means and the valve is opened as necessary to introduce the neutralizing agent into the blood bag (1).

6. The method according to any one of the claims 1 to 4, wherein the blood bag (1) is provided with an air-bleeding tube (10) and the neutralizing agent is introduced into the blood bag (1) from the air-bleeding tube (10).

## Patentansprüche

1. Verfahren für das Inaktivieren eines pathogenen Mikroorganismus unter Verwendung eines Blutbeutelsystems, welches einen Blutbeutel (1), der ein Anticoagulant beinhaltet, einen Behälter (2), der einen Inaktivator beinhaltet, der einen im Blut enthaltenen Mikroorganismus inaktiviert, und ein Verbindungsrohr (5), flüssigkeitsdicht verbunden mit dem Behälter (2), umfasst, wobei der Inaktivator als einen Hauptbestandteil eine Platinverbindung, die an eine Nukleinsäure des Mikroorganismus binden kann, oder einen Aquokomplex der Platinverbindung enthält; das Verfahren umfasst
einen Schritt des Einbringens von Blut in den Blutbeutel (1),
einen Schritt des Einbringens des Inaktivators in den Blutbeutel (1) durch das Verbindungsrohr (5) aus dem Behälter (2), und
gekennzeichnet durch
einen Schritt des Einbringens eines neutralisierenden Mittels in den Blutbeutel (1), um den Inaktivator zu neutralisieren.

2. Verfahren nach Anspruch 1, wobei der Schritt des Einbringens des Inaktivators in den Blutbeutel (1) durch das Verbindungsrohr (5) aus dem Behälter (2) von dem Schritt des Einbringens von Blut in den Blutbeutel (1) gefolgt wird.

3. Verfahren nach Anspruch 1, wobei das Verbindungsrohr (5) mit einem Ventil (51) versehen ist, wobei das Ventil (51) in dem Schritt des Einbringens von Blut in den Blutbeutel (1) geschlossen wird, das Ventil (51) in dem Schritt des Einbringens des Inaktivators in den Blutbeutel (1) durch das Verbindungsrohr (5) aus dem Behälter (2) geöffnet wird.

4. Verfahren nach Anspruch 2, wobei das Verbindungsrohr (5) mit einem Ventil (51) versehen ist, wobei das Ventil (51) in dem Schritt des Einbringens von Blut in den Blutbeutel (1) und in dem Schritt des Einbringens des Inaktivators in den Blutbeutel (1) durch das Verbindungsrohr (5) aus dem Behälter (2) geschlossen wird, das Ventil (51) nach Abschluss des Schritts des Einbringens von Blut in den Blutbeutel (1) und in dem Schritt des Einbringens des Inaktivators in den Blutbeutel (1) durch das Verbindungsrohr (5) aus dem Behälter (2) geöffnet wird.

5. Verfahren nach einem der vorhergehenden Ansprüche, wobei das Blutbeutelsystem mit einem Behälter versehen ist, der das neutralisierende Mittel enthält, welcher mit dem Blutbeutel (1) durch ein Verbindungsrohr verbunden ist, das ein Ventil als ein Öffnungs- und Verschlussmittel hat, und das Ventil nach Bedarf geöffnet wird, um das neutralisierende Mittel in den Blutbeutel (1) einzubringen.

6. Verfahren nach einem der Ansprüche 1 bis 4, wobei der Blutbeutel (1) mit einem Entlüfterrohr (10) versehen ist, und das neutralisierende Mittel in den Blutbeutel (1) aus dem Entlüfterrohr (10) eingebracht wird.

## Revendications

1. Procédé d'inactivation d'un micro-organisme pathogène en utilisant un système de poche de sang qui comprend une poche de sang (1) contenant un anticoagulant, un récipient (2) contenant un inactivateur qui inactive un micro-organisme contenu dans le sang, et un tube de raccordement (5) raccordé de manière étanche au liquide au récipient (2), où l'inactivateur contient comme composant principal un composé de platine capable de se lier à un acide nucléique du micro-organisme ou un complexe aquo du composé de platine ; le procédé comprend
une étape qui consiste à introduire du sang dans la poche de sang (1),
une étape qui consiste à introduire l'inactivateur dans la poche de sang (1) à travers le tube de raccordement (5) à partir du récipient (2), et
**caractérisé par**
une étape qui consiste à introduire un agent de neutralisation dans la poche de sang (1) pour neutraliser l'inactivateur.

2. Procédé selon la revendication 1, dans lequel l'étape d'introduction de l'inactivateur dans la poche de sang (1) à travers le tube de raccordement (5) à partir du récipient (2) est suivie de l'étape d'introduction du sang dans la poche de sang (1).

3. Procédé selon la revendication 1, dans lequel le tube de raccordement (5) est pourvu d'une valve (51), la valve (51) est fermée dans l'étape d'introduction de sang dans la poche de sang (1), la valve (51) est ouverte dans l'étape d'introduction de l'inactivateur dans la poche de sang (1) à travers le tube de raccordement (5) à partir du récipient (2).

4. Procédé selon la revendication 2, dans lequel le tube de raccordement (5) est pourvu d'une valve (51), la valve (51) est fermée dans l'étape d'introduction de sang dans la poche de sang (1) et dans l'étape d'introduction de l'inactivateur dans la poche de sang (1) à travers le tube de raccordement (5) à partir du récipient (2), la valve (51) est ouverte après achèvement de l'étape d'introduction de sang dans la poche de sang (1) et dans l'étape d'introduction de l'inactivateur dans la poche de sang (1) à travers le tube de raccordement (5) à partir du récipient (2).

5. Procédé selon l'une quelconque des revendications précédentes, dans lequel le système de poche de sang est pourvu d'un récipient contenant l'agent de neutralisation qui est raccordé à la poche de sang (1) à travers un tube de raccordement ayant une valve jouant le rôle de moyen d'ouverture et de fermeture et la vanne est ouverte si nécessaire pour introduire l'agent de neutralisation dans la poche de sang (1).

6. Procédé selon l'une quelconque des revendications 1 à 4, dans lequel la poche de sang (1) est pourvue d'un tube de purge d'air (10) et l'agent de neutralisation est introduit dans la poche de sang (1) à partir du tube de purge d'air (10).
